# EUROPEAN PATENT SPECIFICATION

(11) **EP 4 192 571 B1**
(45) Date of publication and mention of the grant of the patent: **12.11.2025**
(21) Application number: 21758640.3
(22) Date of filing: 05.08.2021
(51) Int. Cl.: A61M 60/143, A61M 25/10, A61M 60/295, A61M 60/32, A61M 60/531, A61M 60/585, A61M 60/841, A61M 60/861, A61M 60/867, A61M 25/01, A61M 25/04

(54) **ANCHORED CORONARY SINUS OCCLUSION CATHETER WITH IMPROVED USABILITY**
VERANKERTER KORONARSINUSOKKLUSIONSKATHETER MIT VERBESSERTER NUTZBARKEIT
CATHÉTER D'OCCLUSION ANCRÉ POUR SINUS CORONAIRE À FACILITÉ D'UTILISATION ACCRUE

(30) Priority: 06.08.2020 US 202063062258 P
(43) Date of publication of application: 14.06.2023
(73) Proprietor: Cardiac Impact GmbH, 6340 Baar (CH)
(72) Inventor: HOEM, Jon H., 6315 Oberägeri (CH)
(74) Representative: Hepp Wenger Ryffel AG
(86) International application number: PCT/EP2021/071899
(87) International publication number: WO 2022/029240

(56) References cited:
- EP-B1- 2 389 968
- EP-B1- 2 389 974
- WO-A1-2020/036886
- JP-B2- 5 921 023
- US-A1- 2010 256 506
- US-A1- 2011 295 301
- US-A1- 2019 175 190
- US-A1- 2020 178 974
- US-A1- 2020 206 511
- US-B2- 10 448 884
- US-B2- 8 626 316

## Description

The invention relates to a catheter assembly for the intermittent occlusion of the coronary sinus according to the preamble of the independent claim.

### BACKGROUND

Pressure-controlled Intermittent Coronary Sinus Occlusion (PICSO) (Mohl W, 2015, PICSO: from myocardial salvage to tissue regeneration, Cardiovascular Revascularization Medicine, 36-46) is an evolving technology used to treat patients with microvascular injury after an acute heart attack (ST-segment Elevation Myocardial Infarction, STEMI) (van de Hoef TP, N. F. (2012). Intracoronary Hemodynamic Effects of Pressure-Controlled Intermittent Coronary Sinus Occlusion (PICSO) Results from the First-in-Man Prepare PICSO Study. J Interv Cardiology, 549-556; De Maria, 2018, Index of microcirculatory resistance-guided therapy with PICSO improves coronary microvascular function and reduces infarct size in patients with STEMI the OxAMI-PICSO study. EuroIntervention, e352-359). In addition, the technology shows promise to treat certain types of heart failure (HF) patients (Mohl W, 2018, Acute molecular effects of PICSO in patients with advanved heart failure, ESC Heart Failure, 1176-1183).

The PICSO technology is being applied in the coronary catheterization laboratory ("cathlab") during primary percutaneous coronary intervention (PPCI, also known as "stenting") in case of STEMI patients. For HF patients, the technology may also be applied in the cathlab but without stent implantation as these patients are not in need of coronary revascularization.

PICSO consists of a single use balloon occlusion catheter and a console which controls the inflation and deflation of the balloon by measuring the pressure in the coronary sinus (CS).

The PICSO mode-of-action is still not fully understood. However, it is documented that occlusion of the CS causes a pressure rise throughout the coronary artery vasculature (van de Hoef TP, N. F. (2012). Intracoronary Hemodynamic Effects of Pressure-Controlled Intermittent Coronary Sinus Occlusion (PICSO) Results from the First-in-Man Prepare PICSO Study. J Interv Cardiology, 549-556). As the occlusion of the CS is intermittent, this pressure rise occurs during CS occlusion and is released back to normal anatomic levels when the vessel is opened. It is believed that this cyclical expansion and contraction of the coronary vasculature causes a wash-out effect of the myocardium (like a slushing effect in and out of the microcirculatory bed). This is important as the coronary vasculature potentially is filled by thrombi and other debris as a result of the acute heart attack.

The PICSO technology can be applied in different ways which yield different procedural characteristics. Based on preclinical data (Weigel G, 2007, Beck and back: A paradigm change in coronary sinus interventions - pulsatile stretch on intact coronary venous endothelium. J Thoracic Cardiovasc Surg, 1581-1587), the intermittent occlusion of the CS should be started before opening the infarcted coronary artery. This will create a pressure increase throughout the coronary vasculature which has a potential positive effect on reperfusion injury; i.e. when the coronary artery is opened there is a counter-pressure already created by PICSO in the coronary vasculature from the venous side which counteracts the pressure wave from the arterial blood flow coming into the coronary circulation.

However, due to the current guidelines (Ibanez B, 2018, 2017 ESC Guidelines for the management of acute myocardial in patients presenting with ST-segment elevation, Eur Heart J, 119-177; O'Gara PT, 2013, 2013 ACCF/AHA Guideline for the Management of STEMI. JACC, e78-e140) for the management of STEMI patients, the recommendation is to open the culprit coronary artery immediately after the patient arrives in the cathlab. Therefore, the culprit coronary artery is opened first by passing a guide wire through the coronary lesion or by thrombo-suction or balloon angioplasty (De Maria, 2018, Index of microcirculatory resistance-guided therapy with PICSO improves coronary microvascular function and reduces infarct size in patients with STEMI the OxAMI-PICSO study. EuroIntervention, e352-359). This, in order to re-establish the arterial coronary flow as soon as possible, thereby reducing patient discomfort and reducing further damage to the myocardium ("time-is-muscle"). The opening of the arterial coronary blood flow also serves another purpose as it will increase the CS blood flow thereby making the PICSO procedure more effective (increased arterial flow also increases the coronary sinus outflow).

The cardiologists will now move to the venous side of the coronary circulation. The femoral vein will be opened, and a bi-directional steerable guide sheath is passed from the femoral vein up the inferior vena cava (IVC) and into the right atrium (RA). The bi-directional guide sheaths are typically 8.5F - 12F in inner diameter thereby allowing passage of catheters with outer diameter 2.5 - 4.0mm or up to 8F (Oscor, 2020, *http:*//*www.oscor.com*/*fixed-guiding-sheaths-and-deflectable-steerable-guiding-sheaths*/*destino*/*.* Retrieved from Oscor, Inc.: www.oscor.com). The distal end of the guide sheath is placed into the coronary sinus ostium and the PICSO catheter mounted over a guide wire is advanced through the guide sheath and into the coronary sinus. The guide wire is advanced further into the CS and the 8F PICSO catheter is positioned in the optimal location of the CS, normally in the mid-portion of the CS. The PICSO procedure is now started and the inflation/deflation cycles continued for 30 - 45 minutes.

After the PICSO procedure has been started, the cardiologist will move back to the arterial side and place the coronary stent following standard techniques. The stent placement is normally completed within 5 - 10 minutes so that the PICSO procedure lasts longer than a normal PPCI procedure. Based on recent scientific publications (de Waha S, 2017, Relationship between MVO and adverse events following PPCI for STEMI, Eur Heart J, 3502-3510), 56.9% of STEMI patients, in a meta-analysis of n= 960 patients, have microvascular obstruction post PPCI.

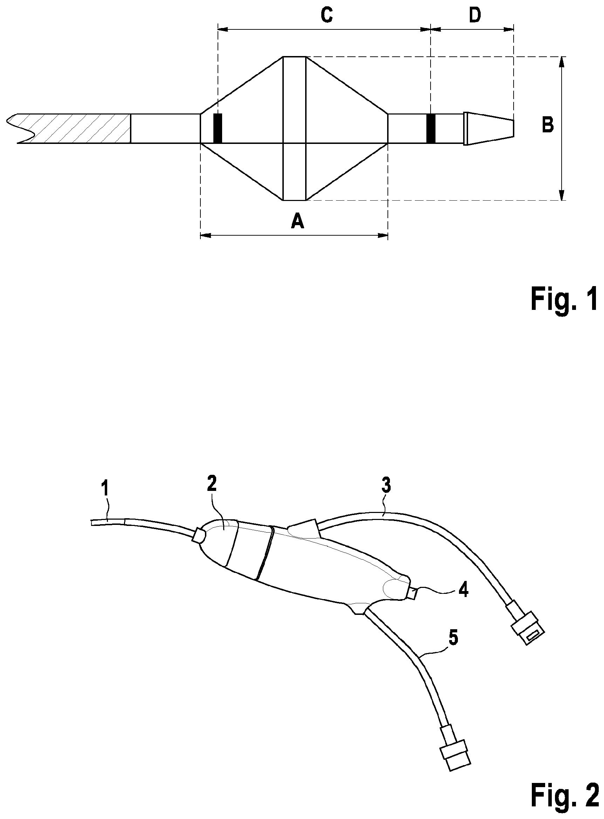

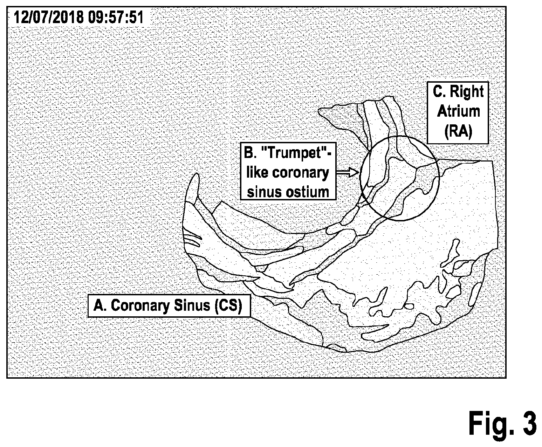

Current prior art PICSO catheters include a distal occlusion balloon (Figure 1), a catheter shaft and a proximal hub or handle (Figure 2). The CS pressure measurement is facilitated through the center fluid-filled line (FFL) lumen. The FFL lumen opening is at the very distal end of the catheter allowing the lumen to be in contact with the CS blood stream. This design is relatively simple to develop and manufacture but has several important drawbacks and limitations:
A. Several steps are needed to deliver the PICSO catheter:
   Due to the coronary sinus location in the right atrium, a bi-directional steerable guide sheath is first passed under angiographic guidance from the femoral vein to engage with the coronary sinus opening (ostium). The PICSO catheter is now advanced over the guide wire, while this assembly is still outside the body, making sure that the distal end of the guide wire protrudes beyond the distal end of the PICSO catheter hub. The PICSO catheter/guide wire assembly is now advanced into the guide sheath. When the assembly exits the guide sheath in the CS ostium, the guide wire is first advanced into the main part of the CS. When the proper position of the guide wire is reached, the PICSO catheter is also advanced into the CS and the proper occlusion balloon location is found by angiographic guidance. The PICSO procedure may now be started by intermittently occluding the coronary sinus by the inflation/deflation of the balloon.
B. The CS occlusion catheter is unstable during CS occlusion: The flow in the coronary sinus is substantial as it drains the total flow from the myocardium. Using a thermodilution technique, Ganz et al. (Ganz W, 1971, Measurement of Coronary Sinus Blood Flow by Continuous Thermodilution in Man. Circulation, 181-195) documented that the average coronary sinus blood flow in humans is 122±25 ml/min with a range of 83 - 159 ml/min. Therefore, and when the occlusion balloon is inflated during PICSO, the force that is exerted on the occlusion catheter is substantial. With the assumption that the human CS ostium diameter is 5-15mm in diameter, an estimate of the force trying to push the balloon out during occlusion is around 0.5N. Even with a stiff catheter shaft using an integrated hypotube and the added support of the guide sheath, recent clinical studies show that the catheter is pushed out in 52% of the placements and in 28% of the cases multiple times. When this occurs, the cardiologists have to "rewire" the coronary sinus by performing the steps described under point A above. This is a nuance and adds time for the patients laying on the hard cathlab table and also adds time to the overall procedure time. During balloon occlusion, there is some support from the balloon pressing against the CS wall. However, the anatomy of the coronary sinus looks like a trumpet (Figure 3) when it enters the right atrium and a uniform force along the occlusion balloon is therefore difficult to obtain. Also, there is a limit for how forceful the balloon can be inflated to avoid coronary sinus wall damage and potential rupture. To reduce the catheter instability, the cardiologists may decide to push the guide wire further into the CS making a bundle at the distal end of the guide wire. Alternatively, the occlusion balloon itself can be placed further into the CS to obtain a more stable position. These corrective measures hold the risk of CS vessel wall damage or vessel rupture. Also, moving the balloon further into the CS reduces the PICSO procedure efficacy as only the vessel tributaries further into the CS from the occlusion balloon position will have an effect from the procedure.
C. The fluid filled pressure lumen does not provide a high-fidelity, low-drift pressure sensing and requires several time-consuming set-up steps:
   Normal length of a coronary sinus occlusion catheter is around 1000 mm) and the fluid filled lumen has a diameter of 20 Gauge equaling 0.81mm. In addition, the fluid-filled lumen is connected to a 1500mm extension line. The fluid-filled lumen has to be flushed before the catheter is introduced into the body and the extension line connected to an external pressure sensor. This external pressure sensor has to be vented to air and connected to the console which controls the balloon inflation. Overall, this results in a long fluid-filled pressure communication to the external pressure sensor which reduces the fidelity of the pressure signal. In addition, flushing the fluid filled pressure lumen, connecting the lumen to the external pressure sensor and then to the console involves many steps and is time-consuming in the cathlab.
D. Proximal handle is not user friendly:
   The current CS occlusion catheters have a proximal handle which is small and difficult to manipulate. As Figure 2 shows, the handle is flat with four connections (the catheter shaft itself, the balloon drive lumen tubing, the balloon pressure lumen and the fluid-filled pressure measurement lumen). There is also no natural fixation of the handle which is problematic as the three console connections (number 3-5 in Figure 2) pull on the catheter shaft (number 1 in Figure 2). To avoid this pulling, the hub needs to be fixat-ed by tape or a tourniquet during the 30 - 45min long procedure.

Document US2020178974 is part of the prior art.

### SUMMARY OF THE INVENTION

The scope of the invention is delimited by the appended set of claims.

It is at least one objective of the present invention to overcome the drawbacks of the prior art. It is in particular an objective to provide catheter assembly that allows a reliable and fast intermittent occlusion of the coronary sinus, easy to operate and showing none or low pressure measurement drift.

The objectives are solved by the subject-matter of the independent claim. Preferred embodiments are described with regard to the dependent claims.

A first aspect of the invention relates to a catheter assembly for the intermittent occlusion of the coronary sinus. The catheter assembly comprises a shaft, the shaft having a plurality of lumens, a distal end with a distal tip, an occlusion device fixed to the distal tip and operable through at least one of the plurality of lumens and a proximal handle. The catheter assembly further has at least one of the following:
- the occlusion device has a diameter of 5 - 15 mm and is adapted to occlude the coronary sinus ostium,
- means of measuring the pressure at the distal tip of the catheter assembly and distally to the occlusion device, preferably using an optical pressure sensor, preferably either on the distal end of the shaft or at the distal end of the anchoring device,
- an anchoring device for anchoring the occlusion device in a predefined position in the coronary sinus, preferably in the ostium,
- the distal end being deflectable/steerable with deflection being controlled by an actuator arranged at the proximal handle.

The term "distal" refers to a position furthers away from the operator. The term "proximal" refers to a position closest to the operator.

The shaft is the main shaft of the device. The shaft can function as a guide sheath.

The smaller diameter of the occlusion device compared to the prior art has a smaller contact area with the tissue of the vessel which allows the positioning of the occlusion device further out in the coronary sinus. This improves the occlusion of the coronary sinus blood flow as more volumetric blood flow will be included, increasing the pressure levels during occlusion and thereby reduces the procedural time.

Means of measuring the pressure at the distal tip of the catheter assembly can be a pressure sensor, preferably an optical pressure sensor and most preferably be connected via an optical cable to the catheter assembly. Means of measuring the pressure can be an optical pressure guidewire with a fibre optic sensor. An electrical pressure sensor is also possible. The pressure sensor can be connected to a console via a pressure sensor cable.

The catheter assembly can be connected to a separate console. Console refers to any device that allows monitoring of the procedure. The console can comprise hardware units such as a computer and a monitor for displaying procedure-relevant information as well as a software for analysis of procedure relevant data, such as pressure-sensitive information.

The advantages of an optical pressure sensor are several and include, but are not limited to:
1. No need to flush the fluid filled lines.
**2.** No calibration required of the sensor.
3. One single connection to the console.
4. The sensor has a small outer diameter (0.26 - 0.64mm) which makes it easier to integrate into distal ends where relatively small areas are available.
5. The sensor comes fully integrated in a nylon sheathing, vinyl catheter tubing and with a SCAI connector. This total assembly is therefore relatively easy to integrate into a catheter design.
6. The SCAI connector is inserted into existing electronic modules which make the integration into a console substantial easier.
7. Wide pressure range: -300 - +300 mmHg 1mmHg=133,322 Pascal
8. Excellent resolution: 0.1 mmHg
9. Excellent system accuracy: ±3 mmHg
10. Low thermal drift: <0.3mmHg/°C

The anchoring device provides stability to the catheter assembly, which avoids that the catheter is pushed out during operation due to pressure arising from the venous blood flow.

A deflectable/steerable distal end allows a simple design of the catheter assembly without the need of additional guidewires. This, in turn, allows a faster procedure as the step of first introducing a guidewire can be omitted.

The plurality of lumens can comprise at least three lumens: a center lumen or inner lumen for accommodating an anchor device, an optical pressure lumen for feeding a pressure sensor cable from the handle to the distal tip and an inflation/deflation lumen for activation and deactivation of the occlusion device. It is also possible to provide four lumens with an additional pressure measurement lumen to monitor the internal pressure of the occlusion device.

The catheter assembly can have only one or more of the above listed features. For instance, instead of the distal end being deflectable and/or steerable, a steerable guidewire could be first placed at a desired treatment site. The guidewire can be arranged within one of the lumens, preferably in a central lumen, and protruding from the distal tip.

Further, the catheter device can comprise an additional outer sleeve, in particular in case of a non-steerable shaft. The outer sleeve may be inserted into the vessel before and may provide guidance to the shaft of the catheter device.

The handle can comprise a deflection actuator and a first locking mechanism for steering and locking the distal tip in a desired configuration. This allows the fixation and securing of the distal tip once it has a desired position or location.

The anchoring device can comprise an anchoring member such as an expandable anchor, preferably consisting of individual expandable members.

"Individual expandable members" can be individual wires, fixed on one or both sides to the shaft. The individual expandable members can be expanded by means of an internal pushing mechanism or can be self-expandable. The anchor can also be connected to a releasable connection line which can be fixed with respect to the shaft.

The anchor or anchoring member can be expanded by moveable proximal members and/or moveable distal members, preferably in the form of markers.

Preferably, the expandable anchor is adapted to be arranged distally to the occlusion device.

The expandable anchor can also be adapted to be arranged proximally to the occlusion device. The expandable anchor can reside proximal to the occlusion device in the right atrium or the inferior vena cava. The expandable members can be pushed open by an internal member in the catheter.

The expandable anchor can also be arranged circumferentially around the occlusion device. The anchor can have a basket-like or cage-like structure.

The anchoring device can comprise an anchoring guidewire, i.e. a guidewire with an integrated anchor forming the anchoring member.

The anchor or anchoring member can have a variety of designs as long as the anchor or anchoring member allows blood to flow through the anchor or anchor member to avoid thrombi.

The shaft can comprise distal and/or proximal markers for angiographic identification of the position of the anchoring device in the coronary sinus. The markers can be arranged in the form of marker bands. The distal marker is preferably located at the distal end of the anchor or at a position showing the furthermost location of the anchor. The proximal marker can be located at the proximal end of the anchor and can move as the anchor is expanded or collapsed.

The actuator of the handle can be formed by a lever for controlling the movement of the distal tip. Preferably, the movement is a bi-directional movement of the distal tip from an unflexed to a flexed position. But the movement can also be a movement in any direction.

Advantageously, the plurality of lumens is encompassed by an outer braided shaft. This provides additional mechanical stability to the lumens.

The catheter assembly can comprise at least one deflection wire, arranged in the outer braided shaft. While the outer braided shaft provides additional mechanical stability, the deflection wire still allows steerability of the distal end.

The distal end can be deflectable up to at least + 90° to -90°. The deflection can be in either direction. The distal end may also be turnable around a central axis, the latter being preferably performed by the operator.

The assembly may comprise a second locking mechanism for axially locking the anchor with respect to the occlusion device, wherein the locking mechanism is preferably a rotational knob located in the centre of the proximal handle.

The locking mechanism can also be located on the outer diameter of the handle. The second locking mechanism can be activated at a distal end of a sheath.

Advantageously, the handle comprises fixation members for a fixed positioning of the handle on a fixed support such as a table during long-term operation of the device. The fixation members can be holes, clamps and the like. The fixation members avoid strain being exerted on the anchor and/or catheter assembly during long-term use.

The occlusion device and/or anchoring device is/are preferably arranged circumferentially around the shaft.

Preferably, the occlusion device is an inflatable balloon. A balloon enables occlusion of the coronary sinus close to the coronary sinus ostium thereby improving the efficacy of the PICSO procedure.

The balloon may be inflatable by helium or air.

The balloon can have an axial length of 5 to 20 mm and/or a diameter of 5 to 20 mm, preferably with a toroidal shape. The dimension provides a shorter landing zone.

By "landing zone" the contact area between balloon and the wall of the vessel is meant. The contact area is smaller than with previous balloon designs, allowing the placement of the balloon closer to the entrance of the coronary sinus. This, in turn, allows a better sealing.

At least two lumens of the plurality of lumen can be adapted to control an expansion and contraction of the occlusion device. For example, the lumen can allow leading a pressurized fluid there through to control inflation or deflation of the balloon.

Current PICSO balloons are inflated and deflated using a closed loop helium filled circuit. In order to optimize the inflation and deflation cycles, the current occlusion balloons have two helium channels. The design consideration is that the deflation speed of the balloon is critical to obtain maximum wash-out from the myocardium after prolonged CS occlusion. Therefore, the balloon deflation is triggered on the ECG cycle so that the balloon deflation is initiated just prior to the next ST-segment. Also, the balloon deflation is achieved by suction of the helium balloon volume out of the balloon.

The current invention does not need to apply suction of the balloon volume for three reasons:
1. Only one inflation/deflation lumen can be used. This makes the catheter and console designs substantially simpler.
2. The balloon can reside further out in the CS and the larger occluding blood flow will collapse the balloon by itself. The ECG triggering may therefore not need and may further simplify the use and design of the console, i.e.: no ECG connections are needed to the patients and the advanced ECG triggering SW algorithm is not needed.
3. The balloon strain in the balloon wall itself can contribute to the collapse of the balloon.

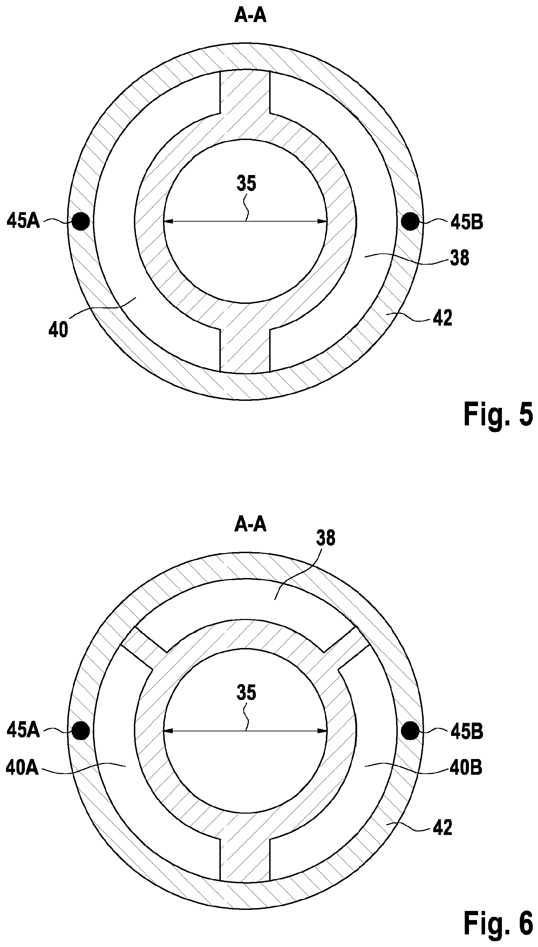

A rapid balloon deflation should occur in a fraction of a heartbeat. Assuming 80 beats/min, one heartbeat is 1.33s long. Of this 1/3 (0.44s) is the systolic portion and the diastolic cycle is 2/3 (0.89s) of the heartbeat. The evacuation and thereby the balloon deflation should therefore be occurring in less than 0.5s to provide optimal wash-out effect of the myocardium. However, if not optimal balloon materials, designs or optimal locations are found then a four-lumen design may be implemented as show in Figure 6.

Preferably, the anchoring device is made of a resilient material, preferably a shape memory material, more preferably nitinol.

Another aspect which is not part of the invention refers to a method for the intermittent occlusion of the coronary sinus. The method comprises the steps of:
a) Placing an anchoring device in a pre-defined location, preferably into the coronary sinus. It is also possible to place the anchoring device in the right ventricle. Preferred is a position where the anchoring device does not affect the outflow from the coronary sinus tributaries.
b) Determining a distance between the anchoring device and a position for placing an occlusion device, preferably in the ostium. Preferably, the optimal distance is determined such as to position the occlusion device such as to occlude as much coronary sinus venous blood outflow as possible.
c) Placing an occlusion device at said position.
d) Fixing the occlusion device in said position by locking the anchoring device to the catheter, preferably by means of a second locking mechanism. The distance between the anchoring device and the occlusion device can thus be fixed.
e) Expanding the occlusion device to occlude the coronary sinus. Expansion is intermittent for intermittent occlusion.

The order of step a) and c) is interchangeable. It is also possible to place first the occlusion device and then the anchoring device.

Advantageously, step a) is preceded by the steps:
f) Advancing a catheter comprising said anchoring device into the right atrium,
g) Directing a distal end of the catheter towards the ostium of the coronary sinus,
h) Locking the distal end in a first configuration by means of a first locking mechanism,
i) Advancing the anchoring device to said pre-defined location.

The anchoring device can be expanded in said pre-defined location.

Further, step e) may be followed by the steps:
j) Collapsing the anchoring device when therapy is completed and intermittent occlusion of the CS ends,
k) Unlocking the second locking mechanism,
l) Withdrawing the anchoring device into the catheter,
m) Unlocking the first locking mechanism to allow free movement of the distal end,
n) Withdrawing the catheter from the right atrium.

Most preferably, the method is performed with a catheter assembly as previously described.

The invention will be described in more details with the aid of figures. The figures describe preferred embodiments and are not to be understood as limiting. They show:
- Figure 1: The engineering drawing of the distal tip of a commercial PICSO catheter.
- Figure 2: Current PICSO catheter proximal hub.
- Figure 3: 3D Echocardiographic image of the coronary sinus.
- Figure 4: An overall catheter design according to the invention.
- Figure 5: A catheter shaft in a cross section along the plane A-A in Figure 4 with three internal lumens.
- Figure 6: An alternative catheter shaft in a cross section along the plane A-A in Figure 4 with four internal lumens.
- Figure 7: View B-B in Figure 4; handle rear end.
- Figure 8: View C-C in Figure 4; distal end with balloon inflated.
- Figure 9: First anchor on distal guidewire with outer sleeve; anchor not deployed.
- Figure 10: Anchor on distal guidewire with outer sleeve according to Figure 9; anchor deployed.
- Figure 11: Second anchor design on distal guidewire without outer sleeve; anchor not deployed.
- Figure 12: Anchor on distal guidewire without outer sleeve according to Figure 11; anchor deployed.
- Figure 13: View A-A of figure 12. The optical pressure sensor (78) may be integrated here or on the guide sheath (28).
- Figure 14: Anchor on guide sheath: "Balloon in cage" design.
- Figure 15: Anchor in guide sheath distal to balloon.
- Figure 16: Anchor on guide sheath proximal to balloon.
- Figure 17: Guide sheath with locking mechanism of guidewire or PICSO catheter either proximal or distal or a combination of the two.
- Figure 18: Anchor of guide sheath in the Right Atrium (RA) or Inferia Vena Cava (IVC).
- Figure 19: Individual anchor wires; balloon in cage solution.
- Figure 20: Individual anchor wires, anchor distal to occlusion device.
- Figure 21: Individual anchor wires; anchor proximal to occlusion balloon.
- Figure 22: Individual anchor wires; anchor in outer sleeve.
- Figure 23: Anchor design without outer sleeve according to the second embodiment (anchor in open position).
- Figure 24: Anchor design without outer sleeve; individual wires and anchor in open position.
- Figure 25: Position of anchor design No. 1-3 in the coronary sinus.
- Figure 26: Potential position of anchor design No. 4 in the coronary sinus.
- Figure 27: Cross section A-A of figure 20 with balloon locking mechanism.
- Figure 28: Cross section A-A of figure 20 with mechanical locking mechanism.
- Figure 29: New vs old occlusion balloon design and location.
- Figure 30: Distal end with different bend radius.
- Figure 31: Flexible distal end of the shaft; cross section.
- Figure 32: Braided wires on the outside of the shaft and covered with an outer sleeve.
- Figure 33: Braided wire in extruded holes.
- Figure 34: Anchor off center and anchor in center.
- Figure 35: The anatomy of the coronary sinus showing the coronary sinus ostium and the emptying of the small and middle cardiac veins close to the ostium.
- Figure 36: The right atrium (RA) anatomy (Guiraudon, G., 2013, Revisiting right atrial isolation rationale for atrial fibrillation functional anatomy of interatrial connections. J Interv Card Electrophysio, 267-273*)* with the inferior vena cava (IVC) and the coronary sinus (CS). The CS ostium is the circular member with openign into the RA**.**

### DESCRIPTION OF EMBODIMENTS

### A. State of the art

Figure 1 shows an engineering drawing of the distal tip of a commercial PICSO catheter. The catheter in size M (Medium) has the following dimensions: the balloon length (A) is 25 mm, the balloon diameter (B) is 15.5 mm, the distance of marker bands (C) is 28.5 mm, the distance of a distal marker band to the balloon-tip (D) is 8.5 mm ± 1.5 mm, the working length is 1000 mm, the extension line length is 1500 mm, the catheter profile is 8.5 F and the maximal guidewire size is 0.032". The catheter in size L (Large) has the following dimensions: the balloon length (A) is 35 mm, the balloon diameter (B) is 20 mm, the distance of marker bands (C) is 33 mm, the distance of the distal marker band to the balloon-tip (D) is 8.5 mm ± 1.5 mm, the working length is 1010 mm, the extension line length is 1500 mm, the catheter profile is 9 F and the maximal guidewire size is 0.032".

Figure 2 shows the current PICSO catheter proximal hub. The hub has the following features: a catheter shaft 1 with strain relief. A hypo-tube ends here in the hub. A drive lumen 3 is connected to the hub 2 for inflating and deflating the PICSO impulse catheter. At the distal end of the drive lumen a drive lumen connector is mounted for connecting to the console. A pressure lumen 5 measures the balloon pressure of the PICSO Impulse Catheter. At the distal end of the pressure lumen is the pressure lumen connector mounted. The hub 4 further comprises a fluid filled line port 4 for the coronary sinus pressure measurement and can be manually flushed with heparinized saline solution. The fluid filled line may be used for applying contrast agent and drawing blood samples. The fluid filled line can be connected with a male luer lock for the extension line to be connected to the console. The current handle has the disadvantages that the many connections to the handle are needed due to the lack of steerability and that the small fish-like handle is difficult to grasp.

Figure 3 shows a 3D Echocardiographic image of the coronary sinus (A) showing the "trumpet"-like coronary sinus Ostium (B) which empties into the right atrium (C).

### B. The Overall Catheter/Sheath Design

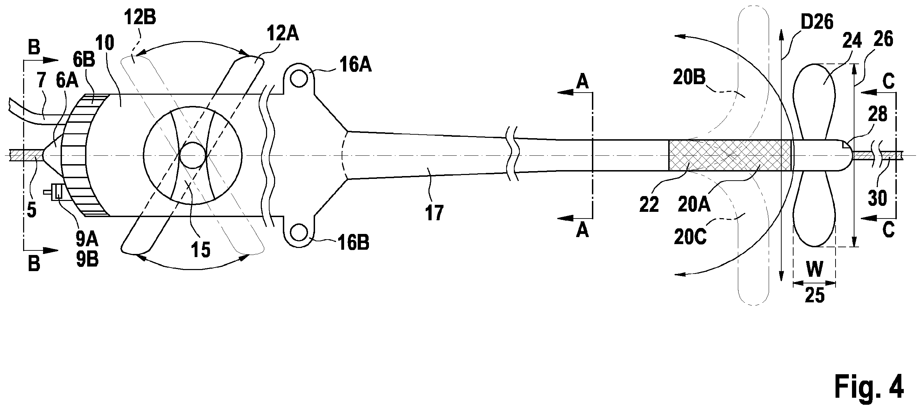

The proposed catheter as shown in Figure 4 shows a handle, bi-directional guide sheath, improved balloon design, integrated optical pressure sensor and a distal guidewire with anchor. The anchor may alternatively be placed directly on the steerable sheath as later figures will show.

In detail, the catheter includes an ergonomic handle (10) with a lever (12A-B) for controlling the flexing of a distal tip (20A-C). The handle contains a first locking mechanism (15) which locks the distal tip in the desired location. In addition, fixation members (16A-B) in the form of holes are integrated on the handle for easier fixed position of the handle while the PICSO therapy is underway. The main shaft (17) of the catheter is tapered and comes down to a size of typically 12 - 16F (4 - 5.4mm) in outer diameter at the distal end.

An occlusion balloon (24) is located directly on the distal tip of the bi-directional catheter and has a different shape than the balloons on current PICSO catheters. The location of the occlusion balloon enables occlusion of the CS close to the CS ostium thereby improving the efficacy of the PICSO procedure. The reason for this improved efficacy is that the small and middle cardiac veins empty close to the CS ostium (see 35). Therefore, the further out in the coronary sinus the occlusion balloon can sit, the more CS blood flow it will occlude.

A right balance between the occlusion balloon location, which influences efficacy, and the correct design and position of an anchor can be determined. This influences safety. As the location of the occlusion balloon will occlude more of the blood volume flow in the CS, there will be a higher force on the balloon during CS occlusion. This force will have to be sustained by the anchor and proper anchor designs and locations are therefore critical.

The balloon design is different from current balloon designs as it occludes the CS ostium with a diameter of 5 - 20 mm and a relative short landing zone, i.e. then zone where it contacts the CS tissue wall Therefore, instead of being an elongated member 25 - 35mm long with an outer diameter of 15.5 - 20mm, this current design has a relatively short width (25) of 5 - 20mm and a diameter (26) of 5 - 20mm.

Only a partial occlusion of the coronary sinus is needed to increase the coronary sinus pressure. An occlusion of 85 - 95% will already give a substantial pressure increase in the coronary sinus.

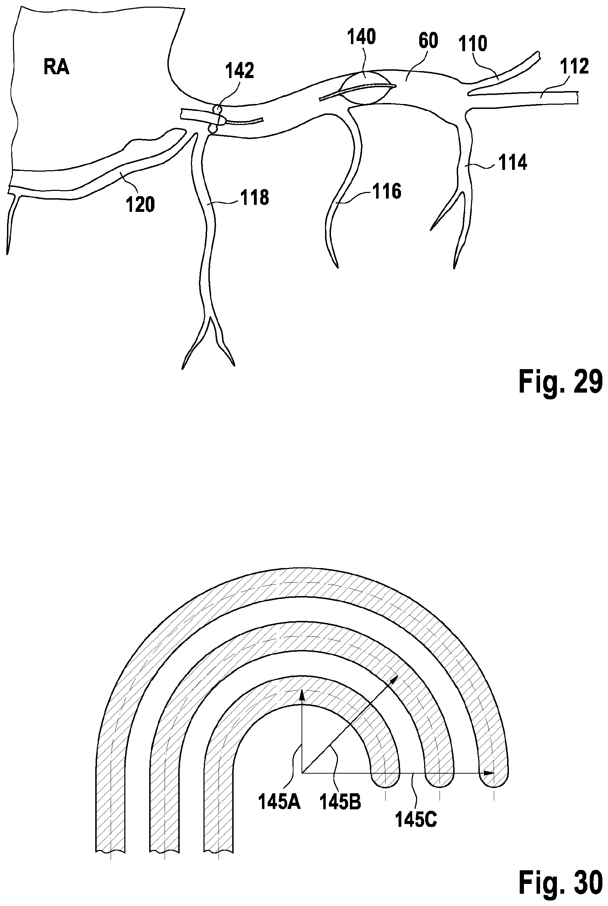

Referring back to Figure 4, the bi-directional movement of the distal tip is controlled by the lever (12A-B) making it possible to continuously flex the distal tip in both directions from an unflexed position (20A) to the flexed positions (20B and 20C). The drawing shows a 90 degrees flex but the design will allow for a flex to 120 degrees and beyond. The balloon (24) may be inflated in all positions but for ease of rendering it is only shown inflated in the straight position (20A). The shaft has a distal flexible portion (22) allowing bending. The flexible portion (22) of the shaft may be manufactured with different lengths to accommodate for a different bend radiuses (see figure 30). This makes the entry into the CS easier depending on the size of the right atrium.

At the handle's proximal end, there are four members: a center lumen from which the anchoring guidewire's (30) proximal end (5)protrudes, a pressure sensor cable (7) which is connected to the console and the balloon inflation/deflation/ measurement lumens (9A-B) also connected to the console.

Alternatively, the pressure sensor (28) may be integrated into the anchoring guidewire (30) in which case only three members protrude from the proximal end.

The three lumen A-A cross-section of the shaft (Figure 5) shows the center lumen (35) which can accommodate the anchoring guide-wire (30, not shown in Figure 5. In this 10F design, the center lumen has inner diameter of 2mm which allows for passing a guidewire with the integrated anchor and, potentially, optical pressure sensor. The optical pressure lumen (38) allows for feeding the pressure sensor cable from the handle to the distal tip. The balloon inflation/deflation lumen (40) passes helium gas from the console to the occlusion balloon. This gas is shuttled back and forth to inflate and deflate the balloon. Finally, an outer braided shaft (42) provides mechanical stability and accommodates the deflection wires (45A-B) to steer the distal end.

In an alternative design, the A-A cross-section has four lumens (Figure 6). The difference from Figure 5 is that the optical pressure lumen (38) is smaller and there are two lumens (40A-B) for controlling the occlusion balloon. The balloon inflation/drive lumen (40A) shuttles the helium gas to and from the balloon. The balloon pressure measurement lumen (40B) monitors the internal balloon pressure. The advantage of this design is that the internal balloon pressure can constantly be monitored for safety reason. This balloon pressure value will also provide important information as the balloon starts to occlude the vessel. As the balloon starts to get closer to the vessel wall, a flutter will be seen in the pressure measurement reading and this will disappear when the balloon is in physical contact with the coronary sinus wall. The vessel occlusion pressure can therefore be easily detected and any additional volume infused into the balloon will expand the balloon and therefore stretch the vessel. Based on experimental and preclinical data, the safe balloon occlusion pressure and volume can therefore be identified before entering clinical trials. In yet another revision of this design, the optical pressure sensor may not be incorporated at all allowing the device to be compatible with existing PICSO consoles. The pressure lumen (38) will then be a fluid-filled line as described above.

In these designs, the inner lumen facilitates the delivery of the anchoring guidewire with or without an optical sensor. The center lumen has an inner diameter of typically at least 2mm. Assuming a 10F compatible sheath, the outer diameter will typically be 4.5 - 5mm leaving the remaining internal area between the center lumen outer diameter and inner diameter of the shaft for the lumens as described above.

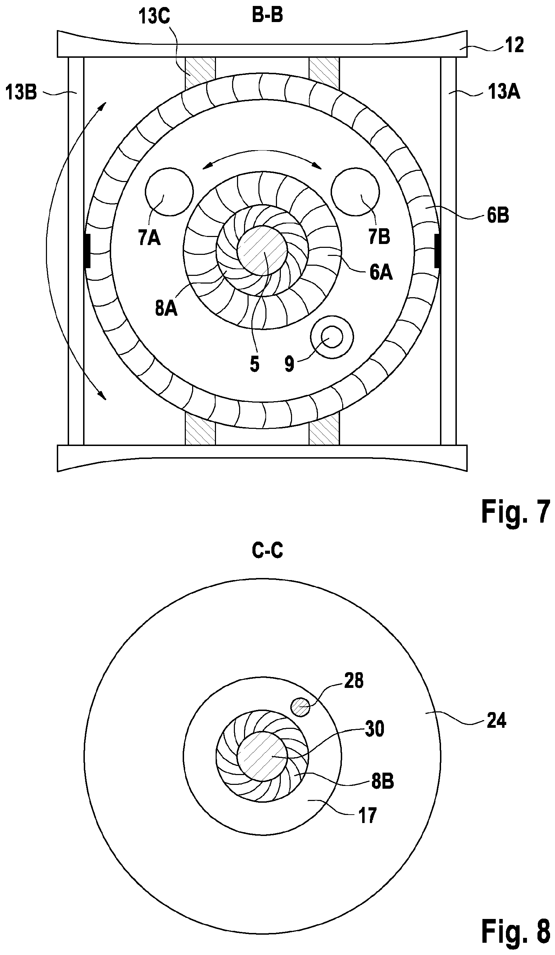

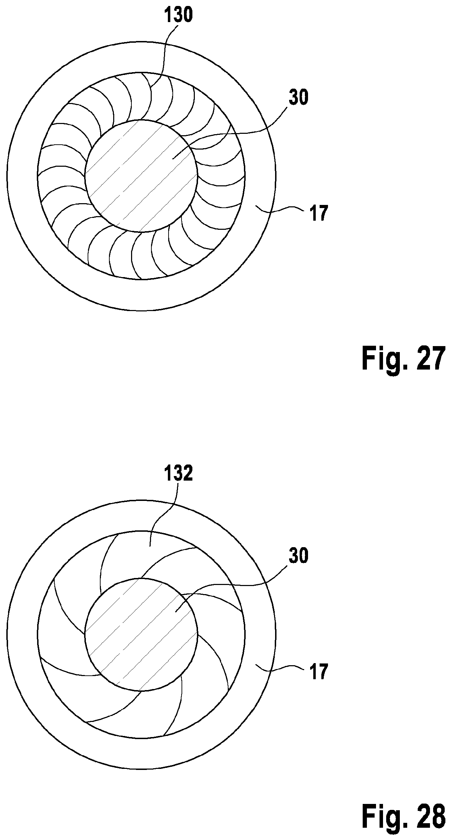

The rear view of the handle (view B-B, Figure 7) shows the features of the proximal end of a guide sheath and handle. In the center, the anchoring guidewire's proximal end (5) is shown which may include the integrated pressure sensor or not (in case if mounted on the sheath itself (28)). The anchoring guidewire can be locked to the sheath itself in order to gain stability. This can be done with a first variant of a second proximal locking mechanism (8A) in the handle itself which is activated by a rotational knob located around the center of the lumen (6A, rotation No. 1) or, alternatively, on the outer diameter of the handle (6B, rotation No. 2). Each rotation is indicated by the curved arrows. As shown in more detail in Fig. 27/28, locking can be achieved by increasing the radial extension of a member arranged coaxially between the anchoring guide wire and the sheath. The member can be e.g. mechanical similar to a shutter or it can be an inflatable balloon.

As shown in Figure 8, a second variant of a second locking mechanism may also be activated at the distal end of the sheath (8B). The locking rotation (6A or 6B) may activate proximal (8A) and distal (8B) locking members as follows:
1. Only the proximal lock (8A)
2. Only the distal lock (8B)
3. The proximal and distal locks (8A and 8B)

The rear end of the handle includes the fiber optical cable output (9) when the pressure sensor (28) is mounted on the distal tip of the flexible shaft. If the pressure sensor is integrated in the anchoring guidewire (30), then the optical cable will exit the assembly through this member. If only one inflation/deflation lumen is used, then the inflation/drive lumen (7A) exits the rear end of the handle. If the balloon inflation/deflation is controlled by two lumens, then the inflation/drive lumen (7A) and the balloon pressure measurement lumen (7B) exit the rear end of the handle.

Figure 7 also shows a lever (12) which controls the flexing of the distal end. This lever is communicating with the first internal mechanism of the handle controlling the deflection wires (45A-B) through the members (13A-C).

View C-C (Figure 8) shows a view from the distal end of the shaft with the balloon (24) inflated. The integrated pressure sensor (28) is also shown as well as the distal end of the anchoring guidewire (30), the distal locking mechanism (8B) and the shaft itself (17). If the pressure sensor is integrated in the anchoring guidewire, then the pressure sensor will not be on the shaft but as an integrated member of the guidewire.

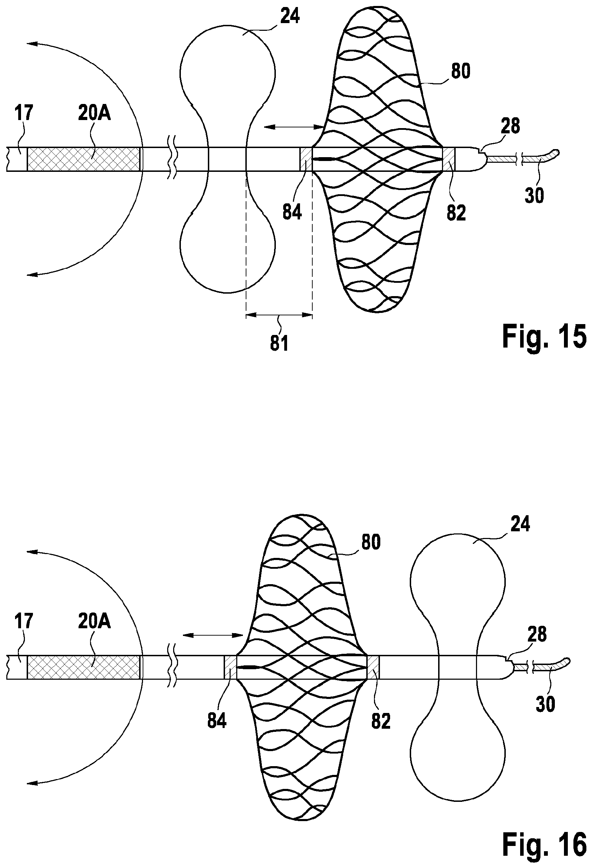

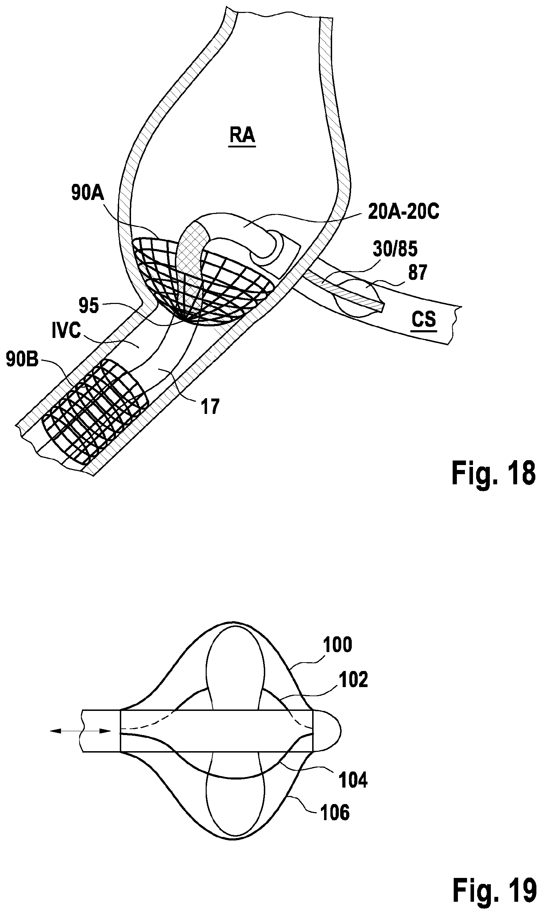

In an alternative and simpler overall design of the invention, a steerable design is foreseen in which no occlusion balloon (24) and no pressure sensor (28) are arranged on the steerable sheath. This variant allows placement of commercially available PICSO catheters and keeping the first locking mechanism in the handle (6A-B and 8A) as well as the second distal locking mechanism (8B). This simpler design may also include anchoring mechanisms of the sheath on the external diameter of the sheath as described in anchor design 6 and 8 (see Figures 16 and 18).

In this simpler overall design, the inner lumen will facilitate the delivery of a commercial guide sheath or PICSO catheter. The center lumen will therefore at least accommodate an 8F PICSO catheter (2.67mm) so that the ID of the sheath typically is not smaller than 3.5mm including the locking mechanism. The sheath outer diameter will then typically be around 14F (4.67mm) but not larger than 16F (5.33mm).

### C. The Optical Pressure Sensor

There are several commercial pressure sensors. For example, the optical pressure sensor can be a FISO optical pressure sensor (FISO Technologies Inc., CA) with a distal end with OD 0.31 mm (FISO, 2020, *https:*//*fiso.com*/*wp-content*/*uploads*/*2018*/*10*/*MC-00263_-Medical-Pressure-Monitoring-Product-Datasheet_R7.pdf.* Retrieved from FISO: www.fiso.com) which are available to be integrated into medical devices. However, none of these have to date been used in a CS occlusion catheter.

As shown in Figure 4, the optical pressure sensor resides distal to the occlusion balloon. This is for safety reasons and to measure the CS pressure during the PICSO procedure. In Figure 4 the sensor is integrated at the tip of the steerable sheath. Alternatively, the sensor may also be integrated into the distal end of the anchoring guide-wire (30) which allows for CS pressure measurements further into the vessel and distal to the anchoring member. This has the advantage that possible clotting of the anchoring member may be detected as well.

### D. The Different Anchor Designs

There are several embodiments for the anchor design. Mainly they can be divided in three groups:
1. Anchors residing in the coronary sinus itself.
2. Locking mechanisms in the guide sheath.
3. Anchors residing in the right atrium or inferior vena cava (IVC).

### i. Anchor Design No. 1: Anchor on Distal Guidewire with Outer Sleeve

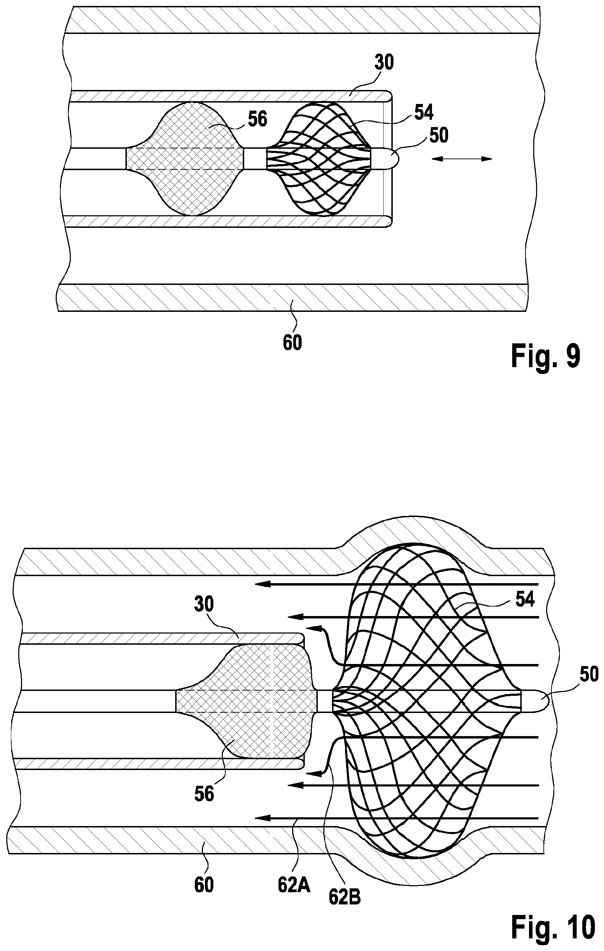

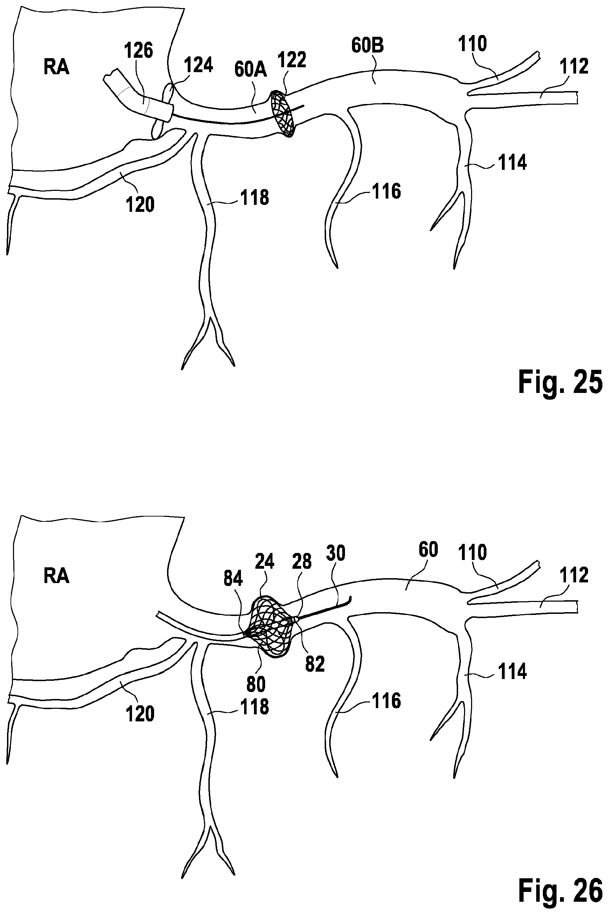

A first anchor embodiment is shown in Figure 9. In this design, the anchor (54) resides in the CS (60) further into the CS from the location of the sheath occlusion balloon and into the main part of the CS as shown in Figure 25.

Further referring to Figure 9, the anchor (54) is mounted on a flexible and atraumatic guidewire (50). Also, on the guidewire (50) and proximal to the anchor (54), a seal or sealing balloon (56) is mounted. The seal or sealing balloon (56) is permanently in contact with the inner diameter of the outer sleeve (30a). This seal or sealing balloon blocks blood from entering the sleeve and acts as a safety mechanism to avoid potential clot- or microthrombi formation.

The anchor (54) itself is shown as a braided member of a re-shapeable material such as nitinol which expands and presses with an adequate force against the CS wall. However, the anchor may use different forms such as individual wires (described below) or other possible expandable embodiments. However, all anchors must allow blood to flow through the anchor when opened and not to cause any thrombi during the overall procedure time.

The guidewire may be advanced forward relative to the outer sleeve (30a) and retracted again into the sleeve after the procedure is completed. Referring to Figure 10, the metal-based anchor deploys when the anchor (54) exits the distal end of the sleeve (30a) and will be compressed when retracted into the distal sleeve again. The blood flow (62A-B) will pass through the anchor. However, due to the seal or sealing balloon (56) the blood will not enter the outer sleeve (30a) and be diverted around the sleeve (62B).

Referring to Figure 25, an alternative anchor design (122) sits in the mid- (60A) to distal (60B) portion of the CS. The figure shows the different main vessels feeding into the CS: the left atrial oblique vein (110), the great cardiac vein (112) emptying blood from the left anterior side of the myocardium, the poster-olateral marginal vein (114), the posterior ventricular vein (116), the middle cardiac vein (118) and the small cardiac vein (120). As shown in the figure, a CS occlusion balloon (124) resides on a steerable guide sheath (126) which sits close the CS ostium. This is an advantage as the occlusion balloon in this position is able to occlude the blood flow from all veins (110 - 120) feeding into the CS (60A-B). This will improve the efficacy of the overall procedure.

This anchor force against the CS wall is strong enough to stabilize the catheter in the CS during balloon occlusion. However, it will not cause intima wall damage, over-stretching nor rupture of the coronary sinus.

Anchor design No. 1 has the advantage that an independent movement between balloon shaft and anchoring device is possible. The anchor can be placed deeper into the coronary sinus. The balloon occludes more coronary sinus blood flow. The anchor can be axially locked with respect to the balloon.

### ii. Anchor Design No. 2: Anchor on Distal Guidewire without Outer Sleeve

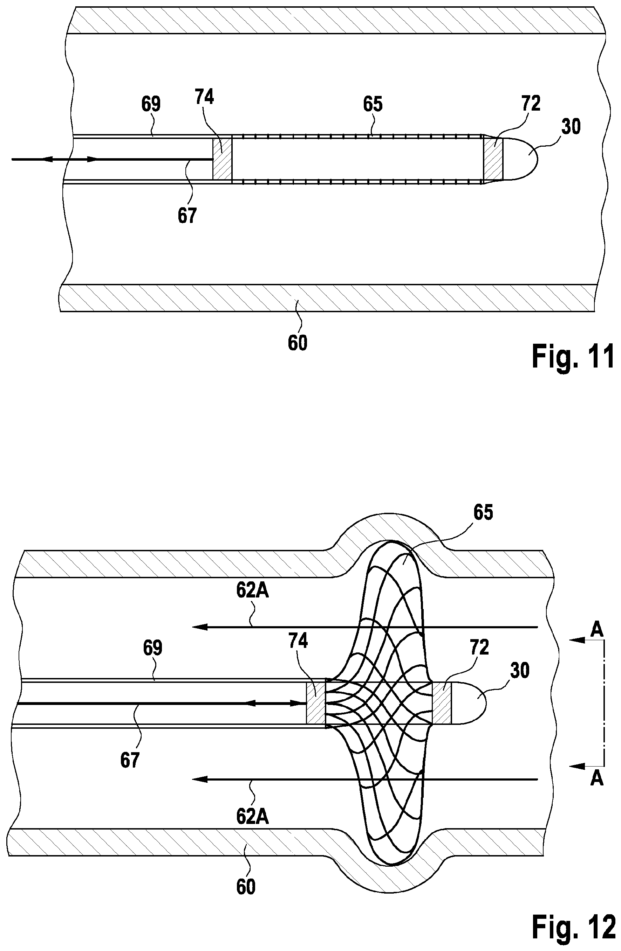

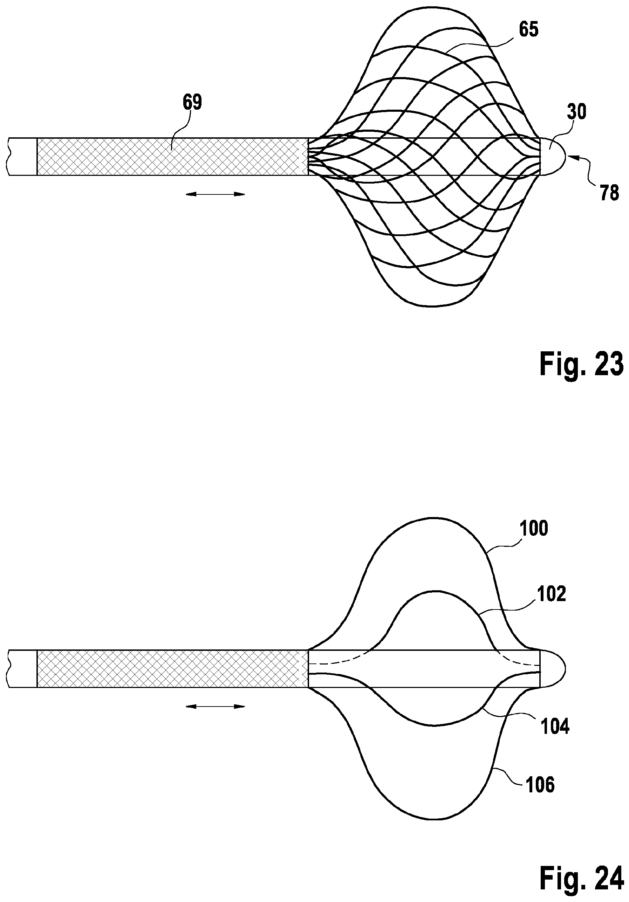

A second anchor embodiment is shown in Figure 11 which shows an anchor (65) on the guidewire in the unexpanded position. This design is simplified to avoid the outer sleeve and only contains the atraumatic guide wire (30) with the expandable anchor (65). This anchor is fixed at the distal end of the guide wire using a soldering ring or fixation element (72). This element (72) may also act as a marker band for angiographic visibility. On the proximal side, the anchor is fixed in a similar manner to a moveable member (74) which also may act as a proximal marker band. The expansion of the anchor is controlled by a moving element (67) inside the guidewire itself which when advanced expands the anchor (65) in the coronary sinus (60) in a controlled manner and under angiographic guidance. Alternatively, the anchor (65) may be expanded by an outer member (69) which opens the anchor when pushed forward. Using this approach, an optical pressure guidewire (78) can pass in the middle of the guidewire. The expanded anchor with this configuration is shown in Figure 23.

Referring to Figure 12, the anchor (65) is expanded and presses against the CS wall (60). A blood stream (62A) will not be restricted to pass the anchor in the expanded position and the anchor will be designed to avoid any thrombi formation of the blood. This can be achieved with different wire coatings on the anchor wires.

Anchor design No. 2 is easier to manufacture than design No. 1 as no sealing balloon is needed. The outer diameter of the guidewire and the anchor size can be optimized.

### iii. Anchor Design No. 3: Optical Pressure Sensor Placed into the Anchored Distal Guidewire

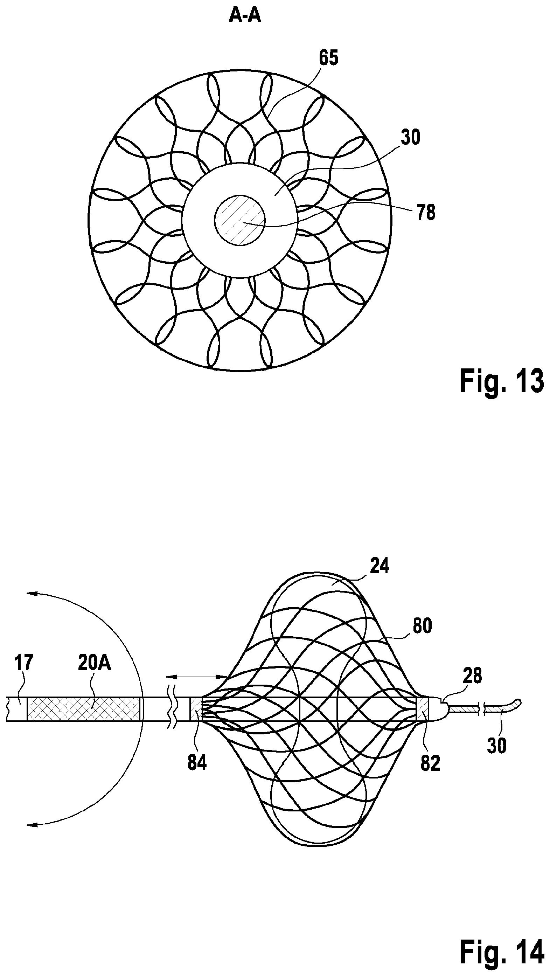

Figure 13 shows view A-A of Figure 12. Here the guidewire main body (30) is shown as well as the expanded anchor. As discussed above, an optical pressure wire (78) is integrated at the distal end of the guidewire. This will allow for pressure monitoring distal to the anchor which may be an advantage to monitor the very distal CS pressure. This is also a safety aspect of this design as potential clotting of the anchor may be detected by the pressure sensor.

This configuration is also shown in Figure 23.

In another configuration and in case the anchor is placed on the guide sheath itself, the guidewire may only include the guide-wire function and the pressure sensor.

Anchor design No. 3 has the advantage that the pressure can be measured distal to the anchor and thus may also detect clotting of the anchor. It reduces the number of wires from the rear part of the handle as the optical wire will exit at the rear end of the anchor/guidewire design.

### iv. Anchor Design No. 4: Anchor on Guide Sheath: Balloon in a Cage

Figure 14 shows an anchor (80) placed on the guide sheath (17) itself.

In this design, the guidewire (30) passes in the center of the guide sheath and the optical pressure sensor (28) is mounted on the distal tip of the sheath.

After placement of the sheath into the coronary sinus, the anchor is expanded by pushing a moveable proximal member (84) which also may be a marker band. On the distal side of the sheath, the anchor is fixed to a non-moving member (82) which also may act as an angiographic marker band.

The anchor (80) is circumferentially arranged around a balloon (24).

When a stable position is found and the CS wall is expanded sufficiently by the anchor, the occlusion balloon (24) may start to intermittently occlude the CS blood flow.

The position of this design will naturally have to be further into the CS as shown in Figure 26.

Anchor design No. 4 has one integrated member, the locking between sheath and anchor is not needed. There is one location for balloon and anchor.

### v. Anchor Design No. 5: Anchor Distal to Balloon (on Guide Sheath)

The anchor of Figure 15 is placed on the guide sheath and in this configuration the anchor is placed distal to the balloon (24). Referring to Figure 15, the anchor (80) is placed close to the distal tip of the steerable guide sheath (17). Again, the anchor may be expanded and collapsed by a moveable member (84) and the fixed member (82) secures the anchor on the distal end. Both these members may be used as angiographic marker bands.

The occlusion balloon (24) sits on the guide sheath proximal to the anchor.

The advantage of this design is that the distance (81) between the anchor and balloon may be adjusted so that the balloon sits closer to the CS ostium thereby occluding more of the CS blood flow.

Anchor design No. 5 also has an integrated member and thus no locking between the sheath and anchor is needed. When there is a small difference in the outer diameter between the shaft and inner diameter of the CS ostium, the relative movement (81) to expand the anchor (80) can be small. This reduces the distal length of the device.

### vi. Anchor Design No. 6: Anchor Proximal to Balloon (on Guide Sheath)

Referring to Figure 16, the balloon (24) is placed distal to the anchor (80). As in the two previous designs, the anchor may be expanded and collapsed by a moveable member (84) and the fixed member (82) secures the anchor at the distal end. Both of these members may be used as angiographic marker bands.

The anchor design No. 6 may allow a better centering of the balloon when procedure starts.

### vii. Anchor Design No. 7: Guide sheath with locking mechanism of guidewire or PICSO catheter either proximal or distal or a combination of the two.

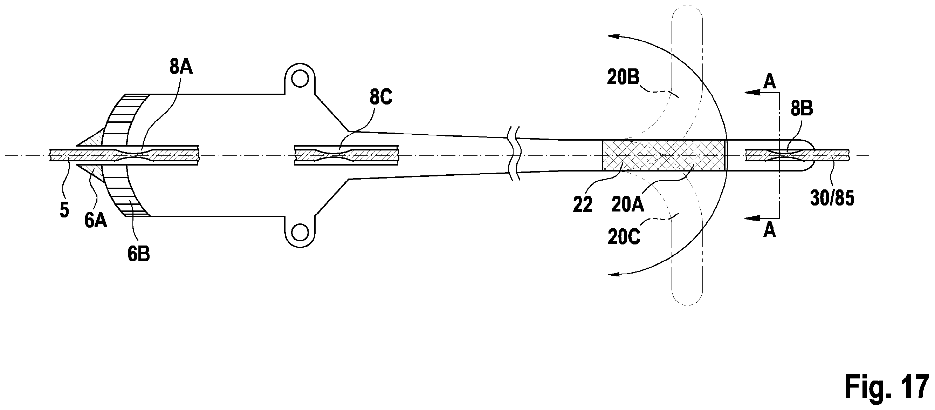

In another implementation (Figure 17) of the anchor, the anchoring effect of the guide wire or a commercial available PICSO catheter is achieved by locking these devices in the guide sheath, or shaft, respectively, itself. This design is for the delivery of these devices only and does not integrate the occlusion balloon or the pressure sensor. However, the guide sheath is bi-directionally steerable as described before (the handle steering mechanism is not shown in Figure 17).

Referring to the same figure, the locking mechanism may be located in the proximal handle (8A and/or 8C) and/or in the distal tip of the sheath (8B). The fixation of the PICSO catheter or guide sheath will likely be most effective the more distal to the tip the locking mechanism is incorporated.

As shown in Figure 27 and Figure 28, the locking mechanism may either be mechanical (like a clamp or shutter) or achieved by a balloon which is inflated around the inner member. The advantage of the latter is that the inner diameter of the shaft (17) may have a balloon like lining which is allowed to expand at certain locations when helium or air is pushed down this lining.

The advantage of the mechanical locking mechanism is that they are more stable than a balloon.

Anchor design No. 7 provides a very simple guide sheath. It is quick and relatively cheap to develop. It can help current PICSO procedures.

### viii. Anchor Design No. 8: Anchor on Guide Sheath in the Right Atrium or Inferior Vena Cava

Referring to Figure 18 showing anchor design No. 8, this solution provides an anchoring mechanism of the sheath completely outside the coronary sinus in the right atrium (RA) or the inferior vena cava (IVC). The RA anchor position (90A) is an expandable metal frame which opens like an umbrella in the RA. The metal frame is fastened with a fixation element (95) proximal to the flexible portion of the shaft. The opening and closing of the umbrella are controlled from the handle of the sheath.

Alternatively, the expandable metal frame (90B) is placed in the IVC and presses in a radial fashion on the inner diameter of this vessel. The opening and closing of this frame are also controlled from the handle.

Anchor design No. 8 does not engage the coronary sinus and lowers the risk of CS complications.

### ix. Anchor Design No. 9: Individual Anchor Wires Instead of Braided Anchor

Finally, and instead of using a braided design of the anchor, the anchor may be designed with 2 - 10 individual wires or more independent wires alongside the shaft. These wires are also memory shaped using materials like nitinol.

Figure 19 shows a balloon in such a cage design with four individual wires (100, 102, 104 and 106). The wires are expanded with a moveable member as described before.

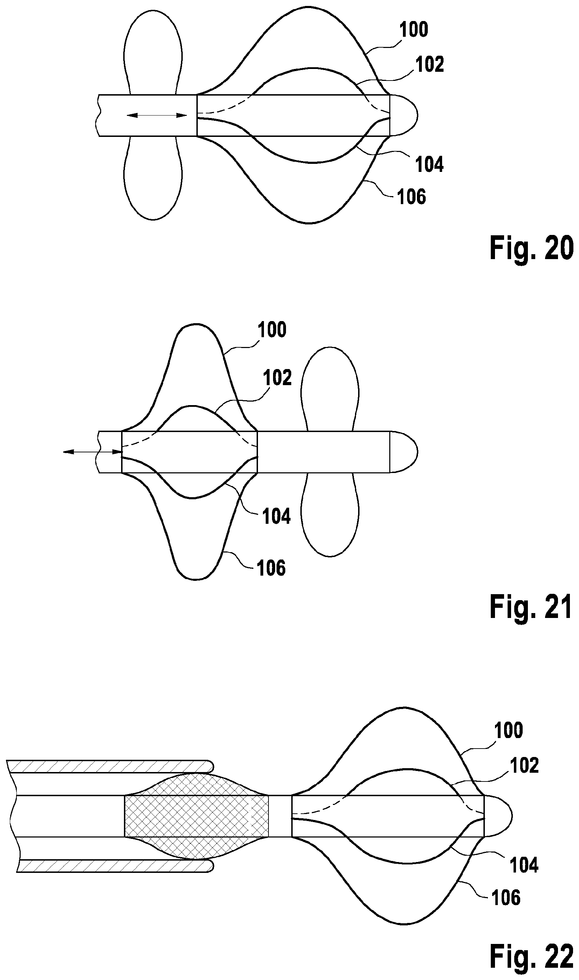

Figure 20 shows a design with an anchor distal to the occlusion balloon again with the four independent wires.

Figure 21 shows a design with a balloon distal to the anchor with four independent wires.

Figure 22 shows the anchor in the outer sleeve design shown in Figure 9 and 10 with the four independent wires and the sealing balloon.

A similar design can be realized on the guide wire without the outer sleeve and balloon as shown in Figure 24.

Anchor design No. 9 is an easy anchor design and easy to manufacture.

Any variation of the anchor designs may of course also be implemented.

### E. Improved Balloon Design

The current PICSO catheters have an elongated balloon with a length of 10 - 15mm. This requires that these balloons (140) are pushed further into the CS to gain stability as shown in Figure 29. This location reduces the efficacy of the PICSO procedure.

The proposed new balloons (142) may be placed further out in the CS thereby occluding more of the CS blood flow. These balloons have a larger diameter (up to 20 - 25mm) than current occlusion balloons and have a short width more suitable to occlude the CS ostium itself. They can be compliant or semi-compliant in design. The balloons may be inflated by hand or automatically from a console.

Referring to Figure 4 and Figure 14, the balloon has a torus shape attached to the shaft (the "neck") over a relative short length typically 3-15mm. The radius of the occlusion balloon, however, can be extended substantially more, typically to 10 - 25mm. The preferred design is that the surface contacting the vessel wall is wider than the neck and enables uniform force distribution along the contacting surface.

In another balloon design, a more pancake shape can be selected to that the neck and contacting surface have more or less the same length.

In both designs the outer diameter (OD) is preferably larger, typically 10 - 20 mm, than the axial length of the balloon.

### F. The Steerable Distal End

The steerable distal end is shown in Figure 30 - Figure 33.

Figure 30 shows different bend radiuses of the distal end typically 30mm (145A), 45mm (145B) or 60mm (145C).

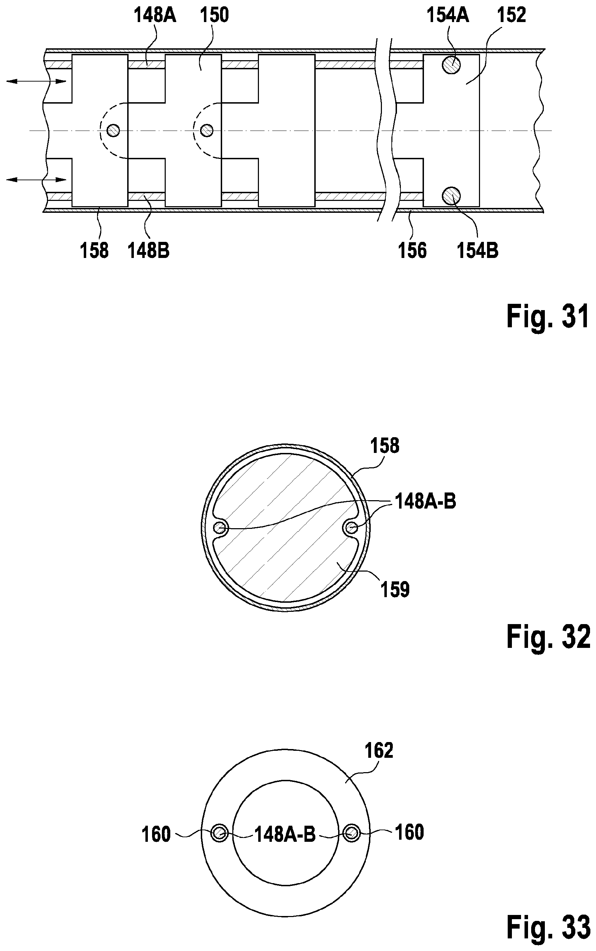

A longitudinal cross-section of the flexible end of the shaft is shown in Figure 31. Braided wires (148A-B), compatible with 45A and 45 B in Figure 5, control the flexing of the distal tip and may be locked in any position using the locking mechanism in the handle. The wires are attached to the last member (152) by soldering or other techniques using the two positions 154A-B. The connecting members (150) are hinged together to facilitate the needed flex of the distal end.

Figure 32 shows the braided wires (148A-B) on the outside of the inner shaft (159). This makes the assembly of the shaft easier as the wires may be placed on the outside of the solid shaft and allows for a larger internal volume. The wires are protected by an outer sleeve (158).

In another design and as shown in Figure 33, the braided wires are fed through extruded holes in the external member (162) of the shaft. This design may be safer but on the expense of the inner volume of the shaft.

### G. The Improved Handle Design

As described in the background of the invention, the current handle designs are not optimized for human manipulation. As shown in Figure 4, there are several new functions which are integrated in the improved handle design:
1) A lever mechanism (12A-B) to steer the distal end (20A-C).
2) A first locking mechanism (15) which can be used to lock the tip in the desired location.
3) The second locking rotation mechanism (6A-B) is used to activate proximal and distal locking members (8A-B in Figure 7andFigure 8). These members are further described and shown in Figure 27and Figure 28 as shutter like radially expandable locking members 130 and 132 and in Figure 17as members 8A-C. If air or helium is used to fixate the PICSO catheter or guidewire, then an inflation port will have to be integrated in the handle as well.
4) Fixation members for the handle as shown in Figure 416A-B.
5) Finally, and as shown in Figure 7, the rear end of the handle provides connections to the balloon inflation lumens (7A-B) and the optical sensor (9). The distal end of the guidewire (5) with or without an optical sensor or anchor is passed through the center lumen of the shaft.
6) For later designs, the following functions may also be incorporated in the handle:
   a. Advance of the anchor to the right position under angiographic guidance
   b. Opening and closing the anchor
   c. Repositioning of the anchor

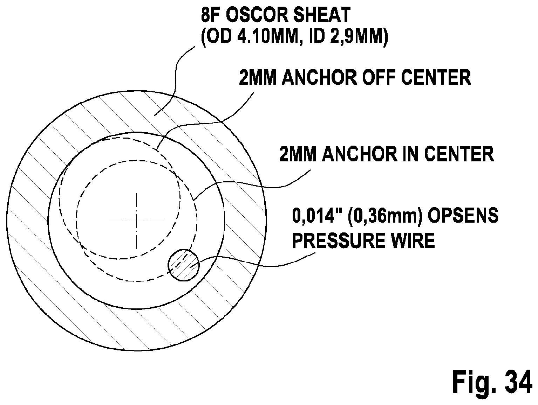

Figure 34 shows an experimental set up for testing the devices in a preclincal model. A commercial available guide sheath such as the 8F Oscor sheath with OD 4.10mm and ID 2.9mm can accommodate a 2mm anchor design with a commercial available OpSens pressure wire with OD 0.36mm. This requires that the anchor is off center for the experimental set-up.

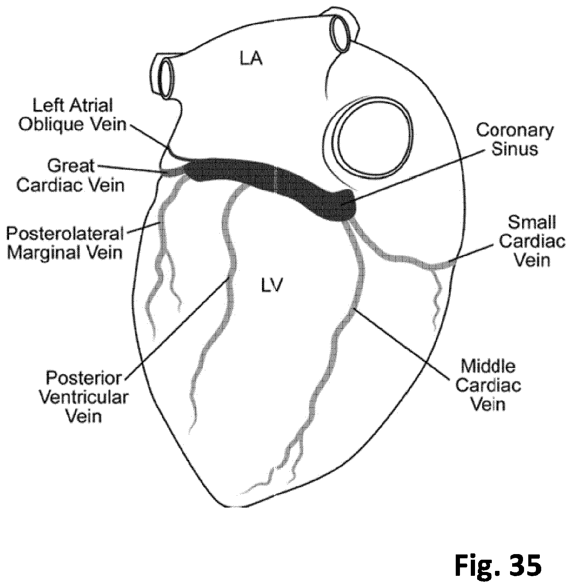

Figure 35 shows the anatomy of the coronary sinus showing the coronary sinus ostium and the emptying of the small and middle cardiac veins close to the ostium as well as the other veins emptying into the CS further into the vessel.

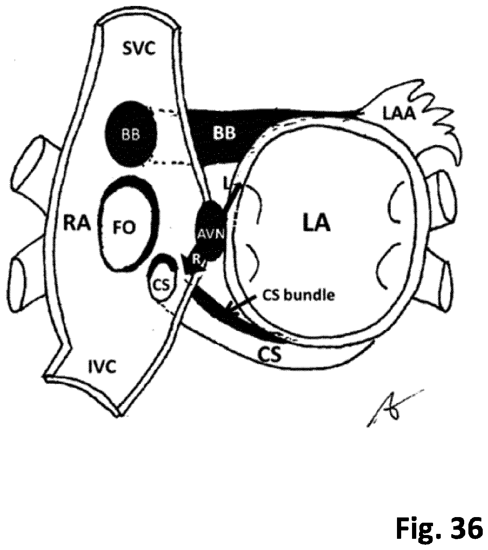

Figure 36 shows a schematic frontal cross section of the atria centered on the circumferential interatrial view. It shows the three interatrial connections crossing over the sulcus: the Bachmann bundle (BB) crosses the upper quadrant, the coronary sinus (CS) bundle crosses the lower quadrant, and the LA-AV nodal connection (left-right) crosses the anterior quadrant. The AV node has a larger expansion to the right and smaller to the left (L). Shown are also the inferior vena cava (IVC), superior vena cava (SVC), fossa ovale (FO), left atrium (LA) and the left atrial appendage (LAA).

## Claims

1. A catheter assembly for the intermittent occlusion of the coronary sinus (CS, 60) comprising a shaft (17, 126), the shaft (17, 126) having a plurality of lumens (7A, 7B, 35, 38, 40, 40A, 40B), a distal end (22) with a distal tip (20A, 20B, 20C), an occlusion device (24, 142) fixed to the distal tip (20A, 20B, 20C) and operable through at least one of the plurality of lumens (7A, 7B, 35, 38, 40, 40A, 40B) and a proximal handle (10), the catheter assembly further having at least one of the following:
- the occlusion device (24, 142) having a diameter of 5 - 20 mm and adapted to occlude the coronary sinus ostium,
- means of measuring the pressure at the distal tip (20 a, 20B, 20C) of the catheter assembly and distally to the occlusion device (24, 142), preferably using an optical pressure sensor (28),
- an anchoring device (30, 54, 65, 80, 90A, 122) for anchoring the occlusion device (24, 142) in a predefined position in the coronary sinus (CS, 60), preferably in the ostium,
- the distal end (22) being deflectable/steerable with deflection being controlled by an actuator arranged at the proximal handle (10).

2. Catheter assembly according to claim 1, wherein the handle (10) comprises a deflection actuator and a first locking mechanism (15) for steering and locking the distal tip (20A, 20B, 20C) in a desired configuration.

3. Catheter assembly according to claim 1 or 2, wherein the anchoring device (30, 54, 65, 80, 90A, 122) comprises an expandable anchor (54, 65, 80, 90A, 122), preferably consisting of individual expandable members (100, 102, 104, 106).

4. Catheter assembly according to claim 3, wherein the expandable anchor (54, 65, 80, 90A, 122) is adapted to be arranged distally to the occlusion device (24, 142).

5. Catheter assembly according to claim 3, wherein the expandable anchor (54, 65, 80, 90A, 122) is adapted to be arranged proximally to the occlusion device (24, 142).

6. Catheter assembly according to claim 3, wherein the expandable anchor (54, 65, 80, 90A, 122) is arranged circumferentially around the occlusion device (24, 142).

7. Catheter assembly according to one of the previous claims, wherein the shaft (17, 126) comprises distal and/or proximal markers (72, 74) for angiographic identification of the position of the anchoring device (30, 54, 65, 80, 90A, 122) in the coronary sinus (CS, 60).

8. Catheter assembly according to claim one of the previous claims, wherein the actuator of the handle (10) is formed by a lever (12a, 12B, 12C) for controlling the movement of the distal tip 20a, 20B, 20C).

9. Catheter assembly according to one of the previous claims, wherein the plurality of lumens (7A, 7B, 35, 38, 40, 40A, 40B) is encompassed by an outer braided shaft (42).

10. Catheter assembly according to claim 9, wherein the catheter assembly comprises at least one deflection wire (45A, 45B), arranged in the outer braided shaft (42).

11. Catheter assembly according to one of the previous claims, wherein the distal end (22) is deflectable up to at least + 90° to -90°.

12. Catheter assembly according to one of claims 3 to 11, wherein the assembly comprises a second locking mechanism (8A, 8B) for axially locking the anchor (54, 65, 80, 90A, 122) with respect to the occlusion device (24, 56, 142), wherein the locking mechanism (8A, 8B) is preferably a rotational knob (6A) located around the centre of the proximal handle (10).

13. Catheter assembly according to one of the previous claims, wherein the handle (10) comprises fixation members (16A, 16B) for a fixed positioning of the handle (10) during long-term operation of the device.

14. Catheter assembly according to one of the previous claims wherein the occlusion device (24, 142) and/or anchoring device (54, 65, 80, 90A, 122) is/are arranged circumferentially around the shaft.

15. Catheter assembly according to one of the previous claims wherein the occlusion device (24, 142) is an inflatable balloon.

16. Catheter assembly according to claim 15, wherein the balloon has an axial length of 5 to 20 mm and/or a diameter of 5 to 20 mm, preferably with a toroidal shape.

17. Catheter assembly according to one of the previous claims wherein at least two lumens of the plurality of lumen (7A, 7B, 35, 38, 40, 40A, 40B) are adapted to control an expansion and contraction of the occlusion device (24, 142).

18. Catheter assembly according to one of the previous claims wherein the anchoring device (30, 54, 65, 80, 90A, 122) is made of a resilient material, preferably a shape memory material.

## Patentansprüche

1. Katheteranordnung zum intermittierenden Verschluss des Koronarsinus (CS, 60), umfassend einen Schaft (17, 126), wobei der Schaft (17, 126) mehrere Lumen (7A, 7B, 35, 38, 40, 40A, 40B) aufweist, ein distales Ende (22) mit einer distalen Spitze (20A, 20B, 20C), eine Okklusionsvorrichtung (24, 142), die an der distalen Spitze (20A, 20B, 20C) befestigt ist und über mindestens eines der mehreren Lumen (7A, 7B, 35, 38, 40, 40A, 40B) betätigt werden kann, und einen proximalen Griff (10), wobei die Katheteranordnung ferner mindestens eines der folgenden Merkmale aufweist:
- die Okklusionsvorrichtung (24, 142) hat einen Durchmesser von 5 bis 20 mm und ist dazu ausgelegt, das Ostium des Koronarsinus zu verschließen,
- Mittel zum Messen des Drucks an der distalen Spitze (20a, 20B, 20C) der Katheteranordnung und distal zur Okklusionsvorrichtung (24, 142), vorzugsweise unter Verwendung eines optischen Drucksensors (28),
- eine Verankerungsvorrichtung (30, 54, 65, 80, 90A, 122) zum Verankern der Okklusionsvorrichtung (24, 142) in einer vordefinierten Position im Koronarsinus (CS, 60), vorzugsweise im Ostium,
- wobei das distale Ende (22) auslenkbar/steuerbar ist, wobei die Auslenkung durch einen am proximalen Griff (10) angeordneten Aktuator gesteuert wird.

2. Katheteranordnung gemäß Anspruch 1, wobei der Griff (10) einen Auskenkungsaktuator und einen ersten Verriegelungsmechanismus (15) zum Steuern und Verriegeln der distalen Spitze (20A, 20B, 20C) in einer gewünschten Konfiguration umfasst.

3. Katheteranordnung gemäß Anspruch 1 oder 2, wobei die Verankerungsvorrichtung (30, 54, 65, 80, 90A, 122) einen expandierbaren Anker (54, 65, 80, 90A, 122) umfasst, der vorzugsweise aus einzelnen expandierbaren Elementen (100, 102, 104, 106) besteht.

4. Katheteranordnung gemäß Anspruch 3, wobei der expandierbare Anker (54, 65, 80, 90A, 122) so ausgelegt ist, dass er distal zur Okklusionsvorrichtung (24, 142) angeordnet werden kann.

5. Katheteranordnung gemäß Anspruch 3, wobei der expandierbare Anker (54, 65, 80, 90A, 122) so ausgelegt ist, dass er proximal zur Okklusionsvorrichtung (24, 142) angeordnet werden kann.

6. Katheteranordnung gemäß Anspruch 3, wobei der expandierbare Anker (54, 65, 80, 90A, 122) umfänglich um die Okklusionsvorrichtung (24, 142) herum angeordnet ist.

7. Katheteranordnung gemäß einem der vorhergehenden Ansprüche, wobei der Schaft (17, 126) distale und/oder proximale Markierungen (72, 74) zur angiographischen Identifizierung der Position der Verankerungsvorrichtung (30, 54, 65, 80, 90A, 122) im Koronarsinus (CS, 60) umfasst.

8. Katheteranordnung gemäß einem der vorhergehenden Ansprüche, wobei der Aktuator des Griffs (10) durch einen Hebel (12a, 12B, 12C) zur Steuerung der Bewegung der distalen Spitze (20a, 20B, 20C) gebildet ist.

9. Katheteranordnung gemäß einem der vorhergehenden Ansprüche, wobei die mehreren Lumen (7A, 7B, 35, 38, 40, 40A, 40B) von einem äußeren geflochtenen Schaft (42) umgeben sind.

10. Katheteranordnung gemäß Anspruch 9, wobei die Katheteranordnung mindestens einen Auslenkungsdraht (45A, 45B) umfasst, der in dem äußeren geflochtenen Schaft (42) angeordnet ist.

11. Katheteranordnung nach einem der vorhergehenden Ansprüche, wobei das distale Ende (22) um mindestens + 90° bis -90° auslenkbar ist.

12. Katheteranordnung nach einem der Ansprüche 3 bis 11, wobei die Anordnung einen zweiten Verriegelungsmechanismus (8A, 8B) zum axialen Verriegeln des Ankers (54, 65, 80, 90A, 122) in Bezug auf die Okklusionsvorrichtung (24, 56, 142) ausweist, wobei der Verriegelungsmechanismus (8A, 8B) vorzugsweise ein Drehknopf (6A) ist, der um die Mitte des proximalen Griffs (10) herum angeordnet ist.

13. Katheteranordnung gemäß einem der vorhergehenden Ansprüche, wobei der Griff (10) Befestigungselemente (16A, 16B) für eine feste Positionierung des Griffs (10) während des Langzeitbetriebs der Vorrichtung umfasst.

14. Katheteranordnung gemäß einem der vorhergehenden Ansprüche, wobei die Okklusionsvorrichtung (24, 142) und/oder die Verankerungsvorrichtung (54, 65, 80, 90A, 122) umfänglich um den Schaft herum angeordnet ist/sind.

15. Katheteranordnung nach einem der vorhergehenden Ansprüche, wobei die Okklusionsvorrichtung (24, 142) ein aufblasbarer Ballon ist.

16. Katheteranordnung nach Anspruch 15, wobei der Ballon eine axiale Länge von 5 bis 20 mm und/oder einen Durchmesser von 5 bis 20 mm aufweist, vorzugsweise mit einer torusförmigen Gestalt.

17. Katheteranordnung nach einem der vorhergehenden Ansprüche, wobei mindestens zwei Lumen der mehreren Lumen (7A, 7B, 35, 38, 40, 40A, 40B) dazu ausgelegt sind, eine Expansion und Kontraktion der Okklusionsvorrichtung (24, 142) zu steuern.

18. Katheteranordnung gemäß einem der vorhergehenden Ansprüche, wobei die Verankerungsvorrichtung (30, 54, 65, 80, 90A, 122) aus einem elastischen Material, vorzugsweise einem Formgedächtnismaterial, hergestellt ist.

## Revendications

1. Ensemble de cathéter pour l'occlusion intermittente du sinus coronaire (CS, 60) comprenant une tige (17, 126), la tige (17, 126) comportant une pluralité de lumières (7A, 7B, 35, 38, 40, 40A, 40B), une extrémité distale (22) avec une pointe distale (20A, 20B, 20C), un dispositif d'occlusion (24, 142) fixé à la pointe distale (20A, 20B, 20C) et pouvant être actionné à travers au moins l'une de la pluralité de lumières (7A, 7B, 35, 38, 40, 40A, 40B) et une poignée proximale (10), l'ensemble de cathéter comportant en outre au moins l'un des éléments suivants :
- le dispositif d'occlusion (24, 142) ayant un diamètre de 5 à 20 mm et étant adapté pour occlure l'ostium du sinus coronaire,
- des moyens pour mesurer la pression à l'extrémité distale (20A, 20B, 20C) de l'ensemble de cathéter et distalement au dispositif d'occlusion (24, 142), de préférence à l'aide d'un capteur de pression optique (28),
- un dispositif d'ancrage (30, 54, 65, 80, 90A, 122) pour ancrer le dispositif d'occlusion (24, 142) dans une position prédéfinie dans le sinus coronaire (CS, 60), de préférence dans l'ostium,
- l'extrémité distale (22) étant défléchissable/orientable, la déflexion étant contrôlée par un actionneur disposé au niveau de la poignée proximale (10).

2. Ensemble de cathéter selon la revendication 1, dans lequel la poignée (10) comprend un actionneur de déflexion et un premier mécanisme de verrouillage (15) pour orienter et verrouiller l'extrémité distale (20A, 20B, 20C) dans une configuration souhaitée.

3. Ensemble cathéter selon la revendication 1 ou 2, dans lequel le dispositif d'ancrage (30, 54, 65, 80, 90A, 122) comprend un ancrage expansible (54, 65, 80, 90A, 122), de préférence constitué d'éléments expansibles individuels (100, 102, 104, 106).

4. Ensemble de cathéter selon la revendication 3, dans lequel l'ancrage expansible (54, 65, 80, 90A, 122) est adapté pour être disposé de manière distale par rapport au dispositif d'occlusion (24, 142).

5. Ensemble de cathéter selon la revendication 3, dans lequel l'ancrage expansible (54, 65, 80, 90A, 122) est adapté pour être disposé de manière proximale par rapport au dispositif d'occlusion (24, 142).

6. Ensemble de cathéter selon la revendication 3, dans lequel l'ancrage expansible (54, 65, 80, 90A, 122) est disposé circonférentiellement autour du dispositif d'occlusion (24, 142).

7. Ensemble de cathéter selon l'une des revendications précédentes, dans lequel la tige (17, 126) comprend des marqueurs distaux et/ou proximaux (72, 74) pour l'identification an-giographique de la position du dispositif d'ancrage (30, 54, 65, 80, 90A, 122) dans le sinus coronaire (CS, 60).

8. Ensemble cathéter selon l'une des revendications précédentes, dans lequel l'actionneur de la poignée (10) est formé par un levier (12a, 12B, 12C) pour contrôler le mouvement de l'extrémité distale (20a, 20B, 20C).

9. Ensemble de cathéter selon l'une des revendications précédentes, dans lequel la pluralité de lumières (7A, 7B, 35, 38, 40, 40A, 40B) est entourée par une tige tressée externe (42).

10. Ensemble de cathéter selon la revendication 9, dans lequel l'ensemble de cathéter comprend au moins un fil de déflexion (45A, 45B), disposé dans la tige tressée externe (42).

11. Ensemble de cathéter selon l'une des revendications précédentes, dans lequel l'extrémité distale (22) peut être défléchie jusqu'à au moins + 90° à -90°.

12. Ensemble cathéter selon l'une des revendications 3 à 11, dans lequel l'ensemble comprend un deuxième mécanisme de verrouillage (8A, 8B) pour verrouiller axialement l'ancrage (54, 65, 80, 90A, 122) par rapport au dispositif d'occlusion (24, 56, 142), dans lequel le mécanisme de verrouillage (8A, 8B) est de préférence un bouton rotatif (6A) situé autour du centre de la poignée proximale (10).

13. Ensemble de cathéter selon l'une des revendications précédentes, dans lequel la poignée (10) comprend des éléments de fixation (16A, 16B) pour un positionnement fixe de la poignée (10) pendant le fonctionnement à long terme du dispositif.

14. Ensemble de cathéter selon l'une des revendications précédentes, dans lequel le dispositif d'occlusion (24, 142) et/ou le dispositif d'ancrage (54, 65, 80, 90A, 122) est/sont disposé(s) circonférentiellement autour de la tige.

15. Ensemble de cathéter selon l'une des revendications précédentes, dans lequel le dispositif d'occlusion (24, 142) est un ballon gonflable.

16. Ensemble de cathéter selon la revendication 15, dans lequel le ballon a une longueur axiale de 5 à 20 mm et/ou un diamètre de 5 à 20 mm, de préférence avec une forme toroïdale.

17. Ensemble de cathéter selon l'une des revendications précédentes, dans lequel au moins deux lumières parmi la pluralité de lumières (7A, 7B, 35, 38, 40, 40A, 40B) sont adaptées pour contrôler l'expansion et la contraction du dispositif d'occlusion (24, 142).

18. Ensemble de cathéter selon l'une des revendications précédentes, dans lequel le dispositif d'ancrage (30, 54, 65, 80, 90A, 122) est constitué d'un matériau élastique, de préférence un matériau à mémoire de forme.
